# FASCICULE DE BREVET EUROPEEN

(11) **EP 3 877 007 B1**
(45) Date de publication et mention de la délivrance du brevet: **05.11.2025**
(21) Numéro de dépôt: 19818223.0
(22) Date de dépôt: 07.11.2019
(51) Int. Cl.: A61L 2/08, B65B 55/08, H01J 37/00

(54) **PROCEDE ET DISPOSITIF DE STERILISATION PAR IRRADIATION D'UN RECIPIENT EN MATIERE THERMOPLASTIQUE**
VERFAHREN UND VORRICHTUNG ZUM STERILISIEREN, DURCH BESTRAHLUNG, EINES BEHÄLTER AUS THERMOPLASTISCHEM MATERIAL
METHOD AND DEVICE FOR STERILIZING, BY IRRADIATION, A CONTAINER MADE OF THERMOPLASTIC MATERIAL

(30) Priorité: 09.11.2018 FR 1860356; 09.11.2018 FR 1860363
(43) Date de publication de la demande: 15.09.2021
(73) Titulaire: Sidel Participations, 76930 Octeville-sur-Mer (FR)
(72) Inventeur: BERNARD, Véronique, 76930 Octeville-sur-mer (FR); CHOMEL, Nicolas, 76930 Octeville-sur-mer (FR); LE PECHOUR, Anthony, 76930 Octeville-sur-mer (FR)
(74) Mandataire: Sidel Group
(86) Numéro de dépôt international: PCT/FR2019/052648
(87) Numéro de publication internationale: WO 2020/094994

(56) Documents cités:
- JP-A- 2002 000 705
- JP-A- H11 248 896
- US-A1- 2018 015 191

## Description

### DOMAINE TECHNIQUE DE L'INVENTION

L'invention concerne un procédé et un dispositif de stérilisation par irradiation d'un récipient en matière thermoplastique.

L'invention concerne plus particulièrement un dispositif selon la revendication 1.

L'invention concerne également un procédé de stérilisation selon la revendication 7.

### ÉTAT DE LA TECHNIQUE

On connaît de l'état de la technique différents procédés de stérilisation pour stériliser au moins l'intérieur d'une préforme et/ou d'un récipient en matière thermoplastique.

La fabrication d'un récipient en matière thermoplastique est obtenue à partir d'une préforme chaude, généralement préalablement conditionnée thermiquement dans un four d'une installation de fabrication de récipients avant d'être introduite dans un moule pour y être transformée par soufflage au moyen d'au moins un fluide sous pression, avec ou sans étirage.

On fabrique ainsi différents types de récipients (bouteilles, flacons, pots, etc.) qui sont notamment, mais non exclusivement, destinés à être utilisés pour le conditionnement de produits dans l'industrie agro-alimentaire.

Dans le domaine de la fabrication de récipients pour l'industrie agro-alimentaire, on recherche par tous moyens à réduire les risques de contaminations microbiologiques des récipients par des agents pathogènes, soit des micro-organismes.

C'est la raison pour laquelle, la Demanderesse a déjà proposé de mettre en œuvre différentes actions pour éliminer des agents pathogènes, tels que les germes (bactéries, moisissures, etc.), qui sont susceptibles d'affecter le produit contenu dans de tels récipients.

On peut en particulier distinguer d'une part les actions visant à détruire les micro-organismes pour stériliser au moins l'intérieur du récipient et, d'autre part, les actions visant plus généralement à prévenir la contamination des récipients par de tels micro-organismes.

Les documents de l'état de la technique FR-2.915.127, WO-03/084818 et EP-2.094.312, auxquels on se reportera pour de plus amples détails, sont cités à titre d'exemples non limitatifs de telles actions.

Le document FR-2.915.127 décrit une installation de fabrication de récipients comportant une enceinte de protection délimitant une zone à l'intérieur de laquelle est agencée une machine de moulage de récipients de type souffleuse qui est alimentée par des moyens de transfert en préformes préalablement conditionnées thermiquement dans un four.

Selon les enseignements de ce document, l'installation comporte un système d'insufflation d'air filtré à l'intérieur de l'enceinte pour y établir notamment une surpression de manière à limiter les risques de contamination tant des préformes en sortie du four que des récipients fabriqués.

Le document WO-03/084818 décrit par exemple un traitement de décontamination par irradiation du col de préformes par un rayonnement de type ultraviolet (UV), avant l'introduction des préformes dans le four.

Le document EP-2.094.312 décrit par exemple un traitement par irradiation avec un rayonnement ultraviolet (UV) mis en œuvre de manière particulière dans un four pour décontaminer au moins la surface externe de la préforme en cours de conditionnement thermique.

Le document WO-2006/136498 au nom de la Demanderesse décrit par exemple un traitement de décontamination d'une préforme consistant à déposer par condensation un film de buée sensiblement uniforme d'un agent stérilisant sur la paroi interne de la préforme.

Un tel traitement de décontamination par condensation, dit par « voie chimique », donne satisfaction puisque des degrés de décontamination jusqu'à 6Log sont obtenus.

On rappelle que la quantité de micro-organismes est susceptible d'être dénombrée par comptage après notamment des opérations de lavage, de filtration et de mise en culture.

On détermine ainsi une réduction logarithmique du nombre de micro-organismes par exemple dite de l'ordre de 3Log (ou encore 3D) équivalent à 1000 unités (10³).

Cependant, on recherche des solutions alternatives à la décontamination par voie chimique permettant de ne pas utiliser d'agent stérilisant, comme le peroxyde d'hydrogène (H2O2), mais sans toutefois sacrifier pour autant au résultat obtenu pour la décontamination.

Dans le document WO-2016/120544, la Demanderesse a proposé une solution alternative consistant à procéder à la stérilisation d'un récipient en matière thermoplastique au moyen d'un faisceau d'électrons pulsé et d'un réflecteur mobile.

Pour l'application industrielle d'un tel procédé de traitement pour la stérilisation de récipients, outre la maîtrise technique, l'un des enjeux est aujourd'hui essentiellement d'ordre économique, en raison du coût élevé d'un émetteur utilisé pour obtenir le faisceau d'électrons. D'autres documents traitant de la stérilisation des récipients à l'aide de faisceaux d'électrons sont JP H11 248896 A, JP 2002 000705 A et US 2018/015191 A1.

C'est la raison pour laquelle, on recherche des solutions permettant d'optimiser l'utilisation d'un émetteur, de réduire le nombre total d'émetteurs, et cela tout en ayant les mêmes résultats de stérilisation des récipients.

Or, il est nécessaire d'avoir une durée d'irradiation qui soit suffisante lors du traitement pour irradier les surfaces du récipient à stériliser avec une quantité d'électrons permettant d'obtenir une dose létale pour les micro-organismes, c'est-à-dire une dose supérieure ou égale à 10 kGy (kilo-Gray).

Le procédé de traitement doit également être compatible avec les cadences actuelles de production de récipients qui atteignent par exemple, dans le cas de bouteilles en PET, plus de 60.000 bouteilles par heure.

Le but de l'invention est notamment de proposer un nouveau dispositif de stérilisation permettant de résoudre au moins une partie des inconvénients de l'état de la technique et tout particulièrement d'optimiser l'utilisation d'un émetteur pour chaque type de récipients et de réduire le nombre d'émetteur(s).

### BREF RESUME DE L'INVENTION

Dans ce but, l'invention propose un dispositif de stérilisation du type décrit précédemment, caractérisé en ce que ledit au moins un émetteur est configuré pour pouvoir incliner la tête suivant un angle d'inclinaison qui, compris entre l'axe principal de l'émetteur et l'axe du récipient, est déterminé en fonction de la hauteur du récipient afin d'optimiser la dose d'irradiation reçue par ledit récipient.

Selon l'invention, le récipient est irradié par un faisceau d'électrons avec une dose qui est supérieure ou égale à celle de l'art antérieur lorsque ledit récipient présente une hauteur inférieure à celle de la tête de l'émetteur, c'est-à-dire une dose létale pour les micro-organismes.

Avantageusement, ladite dose létale est susceptible d'être obtenue avec un dispositif de stérilisation comportant moins d'émetteur(s) en raison de l'augmentation de la durée d'irradiation, de la maximisation de la dose d'irradiation reçue.

Avantageusement, l'invention permet de réduire le nombre d'émetteurs nécessaires grâce à l'augmentation de la durée d'irradiation des récipients, l'optimisation de la dose d'irradiation délivrée par la tête de l'émetteur pour un récipient d'une hauteur donnée et cela sans toutefois réduire au final la cadence, c'est-à-dire la vitesse moyenne de circulation des récipients.

Avantageusement, la mise en œuvre d'un procédé de traitement selon l'invention est compatible avec les cadences de fabrication de récipients et donc susceptible de recevoir une application industrielle en intégrant un tel dispositif de stérilisation dans une installation de fabrication de récipients, tels que des bouteilles en PET.

Avantageusement, la stérilisation du récipient est effectuée en irradiant un récipient vide, avant de procéder au remplissage.

De préférence, l'irradiation du récipient est mise en œuvre dans une installation de fabrication de récipients entre l'unité de moulage (ou souffleuse) et l'unité suivante, tel qu'une unité de remplissage ou d'étiquetage.

Selon les applications, un étiquetage des récipients est en effet susceptible d'être réalisé avant ou après leur remplissage.

Par comparaison avec un procédé de décontamination de préformes par voie chimique selon le document WO-2006/136498 précité, l'invention permet de simplifier grandement la conception d'une installation de fabrication de récipients à partir d'une préforme, en particulier l'unité de moulage (ou souffleuse).

La stérilisation du récipient final (et non de la préforme) permet de s'affranchir de nombreux moyens jusqu'alors mis en œuvre dans une telle installation de fabrication de récipients, les micro-organismes présents étant détruits lors de l'irradiation du récipient au moyen du faisceau d'électrons, préférentiellement de type pulsé.

Ainsi, il n'est plus nécessaire de mettre en œuvre des moyens spécifiques (tels que des systèmes d'insufflation, etc.) pour préserver la stérilité d'une préforme postérieurement à son traitement chimique, c'est-à-dire au cours de son conditionnement thermique, de sa transformation par soufflage ou étirage-soufflage en récipient et cela jusqu'au remplissage et à la fermeture du récipient.

Avantageusement, la stérilisation par irradiation selon l'invention permet de stériliser simultanément tant l'intérieur que l'extérieur d'un récipient.

Ainsi, les dispositifs de traitement des préformes par irradiation au moyen d'un rayonnement UV sont susceptibles d'être supprimés.

Les systèmes d'insufflation et plus généralement de filtration de l'air participant à l'obtention d'un environnement de fabrication propre sont également susceptibles d'être supprimés.

Avantageusement, la suppression de l'ensemble de tels dispositifs et/ou systèmes permet de réaliser des économies importantes sur le coût d'une installation de fabrication et cela tant à l'acquisition qu'à l'exploitation.

Avantageusement, l'étape d'irradiation étant réalisée en aval de l'unité de moulage (ou souffleuse) dans une installation de fabrication, les actions réalisées jusqu'alors en amont de l'unité de moulage, notamment en vue de la destruction des micro-organismes et de la prévention des risques de contamination des récipients, peuvent être en tout ou en partie supprimées.

Avantageusement, la conception de l'unité de moulage (ou souffleuse) s'en trouve particulièrement simplifiée et son coût de fabrication comme d'exploitation réduit. En effet, les opérations de nettoyage dites « CIP » (acronyme pour « Clean-in-Place » en anglais) peuvent être supprimées.

Par conséquent, il n'est plus nécessaire de recourir pour l'unité de moulage à l'utilisation de matériaux coûteux, tels que l'inox, choisis pour leur résistance aux agressions chimiques, notamment la corrosion, résultant des produits de nettoyage utilisés lors de telles opérations de « CIP ».

Avantageusement, on appréciera que l'invention va à l'encontre des enseignements de l'état de la technique consistant, pour préserver la cadence, à multiplier le nombre d'émetteurs le long du parcours jusqu'à atteindre une dose d'irradiation suffisante pour stériliser chaque récipient.

### Selon d'autres caractéristiques de l'invention :

- l'angle d'inclinaison de la tête est déterminé de manière que le ratio de la hauteur du récipient sur la hauteur de l'émetteur soit proche de 1 ;
- l'angle d'inclinaison de la tête est compris entre une valeur minimale supérieure à 0° et une valeur maximale de 90° ;
- la tête de l'émetteur est montée mobile en rotation afin d'incliner la tête suivant ledit angle d'inclinaison ;
- l'inclinaison de la tête est commandée par des moyens d'actionnement ;
- la tête est montée mobile en translation pour faire varier la distance entre la tête et ledit au moins un récipient à irradier ;
- la tête est télescopique par rapport à l'émetteur ;
- l'émetteur est monté mobile en translation relativement audit au moins un récipient à irradier.

L'invention propose encore un procédé de stérilisation du type décrit précédemment, caractérisé en ce que, la direction de déplacement suivi par le flux de récipients transporté par un système de convoyage étant orthogonale à l'axe principal des récipients, ledit procédé de traitement comporte au moins :
- une étape d'irradiation dudit au moins récipient depuis l'extérieur par un faisceau d'électrons émis par la tête dudit émetteur agencée pour présenter un angle d'inclinaison qui, compris entre l'axe principal de l'émetteur et l'axe du récipient, est égal à une valeur déterminée en fonction de la hauteur du récipient.

Avantageusement, ledit procédé de traitement comporte au moins une étape préliminaire de réglage consistant, lorsque la direction de déplacement suivi par le flux de récipients est orthogonale à l'axe principal des récipients, à régler l'inclinaison de la tête dudit émetteur suivant un angle d'inclinaison d'une valeur déterminée pour que le ratio de la hauteur du récipient sur la hauteur de l'émetteur soit proche de 1.

Avantageusement, ledit flux de récipients étant transporté par le système de convoyage suivant un parcours donné, avec un écartement déterminé, dit pas initial, correspondant à la distance entre les axes de deux récipients consécutifs, le procédé de traitement comporte au moins une étape de commande du système de convoyage pour faire varier sélectivement ledit pas initial entre deux récipients consécutifs afin de réduire, au moins dans une zone d'irradiation du parcours dans laquelle est agencé ledit au moins émetteur comportant la tête, l'écartement entre les récipients dudit flux à un pas proximal qui est inférieur audit pas initial.

### BREVE DESCRIPTION DES FIGURES

D'autres caractéristiques et avantages de l'invention apparaitront au cours de la lecture de la description détaillée qui va suivre pour la compréhension de laquelle on se reportera aux dessins annexés dans lesquels :

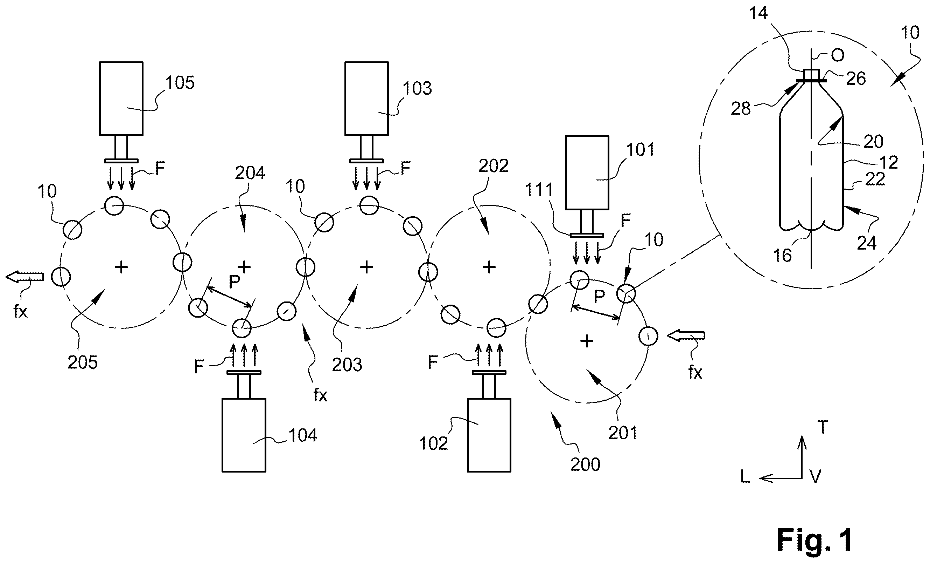

- la figure 1 est une vue de dessus qui représente schématiquement des récipients en matière thermoplastique, par exemple ici des bouteilles, formant un flux et qui sont transportés avec un pas constant par un système de convoyage suivant un parcours le long duquel sont agencés des émetteurs d'un dispositif de stérilisation destinés à irradier lesdits récipients depuis l'extérieur avec un faisceau d'électrons et qui illustre la mise en œuvre d'un traitement pour la stérilisation par irradiation de récipients selon l'état de la technique ;

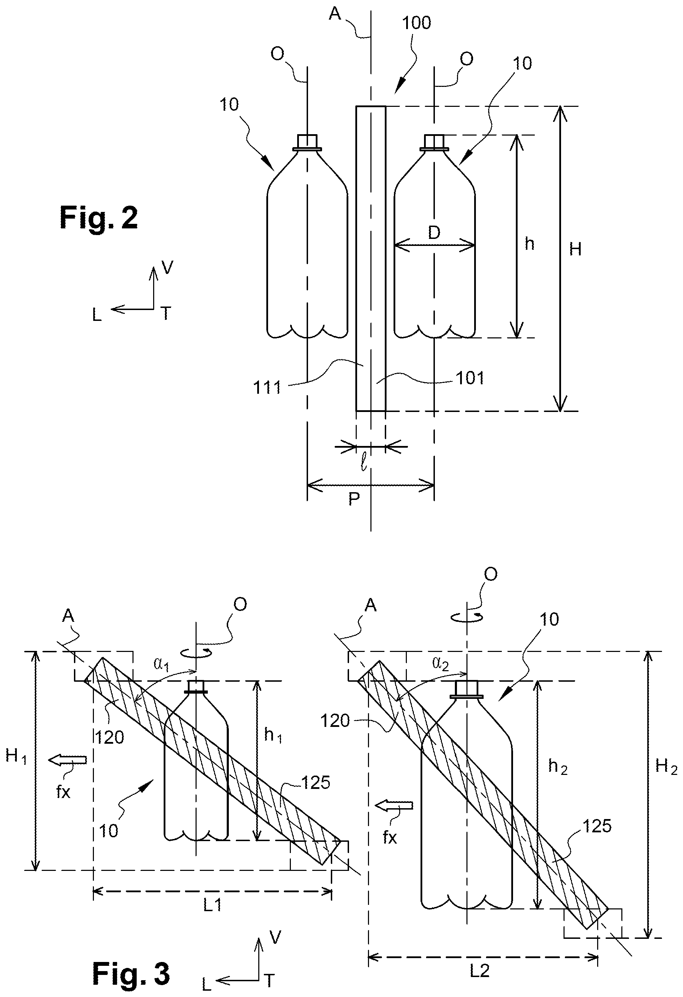

- la figure 2 est une vue de côté qui représente une tête d'un des émetteurs du dispositif de stérilisation selon l'état de la technique de la figure 1 qui est agencé sur un tronçon de parcours pour former une zone de stérilisation par irradiation des récipients et en vis-à-vis de laquelle se trouve deux récipients consécutifs du flux et qui illustre ladite tête dont l'axe A est coaxial à l'axe O des récipients ;
- la figure 3 est une vue de côté qui, analogue à la figure 2, représente un émetteur comportant une tête qui est inclinée selon les enseignements de l'invention d'un angle d'inclinaison déterminé en fonction des dimensions du récipient, en particulier la hauteur, et qui illustre une telle inclinaison de la tête de l'émetteur par rapport à l'axe du récipient selon un angle d'inclinaison déterminé pour optimiser l'irradiation dudit récipient d'une hauteur donnée, respectivement pour deux récipients de dimensions différentes.

### DESCRIPTION DETAILLEE DES FIGURES

Dans la suite de la description, on adoptera à titre non limitatif les orientations longitudinale, verticale et transversale en référence au trièdre (L, V, T) représenté sur les figures.

Par convention, les orientations longitudinale et transversale définissant ensemble un plan horizontal sont déterminées de manière fixe par rapport aux récipients.

On utilisera à titre non limitatif les termes « amont » et « aval » en référence au sens de déplacement des récipients qui sont transportés par le système de convoyage de l'amont vers l'aval suivant un parcours donné.

On utilisera également à titre non limitatif les termes « supérieur » et « inférieur » ou « haut » et « bas » en référence à l'orientation verticale. Les termes « intérieur » ou « extérieur » et « interne » ou « externe » sont notamment utilisés par rapport aux récipients, le volume intérieur étant délimité par la paroi du récipient dont le corps est muni d'un col et fermé par un fond de sorte que ladite paroi délimite respectivement l'intérieur et l'extérieur.

Dans la description qui suit, les éléments désignés par les mêmes chiffres de référence désignent des moyens analogues, similaires ou identiques.

On a illustré, sur les figures 1 et 2, un dispositif 100 de stérilisation pour la mise en œuvre d'un procédé de traitement pour la stérilisation par irradiation de récipients 10 selon l'état de la technique pour la Demanderesse.

Dans la présente demande, les récipients 10 en matière thermoplastique à stériliser sont préférentiellement des bouteilles, notamment en PET (Polyéthylène Téréphtalate), fabriquées à partir d'une préforme. En variante, le récipient 10 en matière thermoplastique est un bidon, un flacon, un pot, etc.

Tel qu'illustré sur la figure 1, le récipient 10 comporte un corps 12 muni d'un col 14 et fermé par un fond 16, ledit col 14 délimitant circonférentiellement une ouverture.

Le récipient 10 comporte une surface 20 interne délimitée par une paroi 22 formant le corps 12, le col 14 et le fond 16, ainsi qu'une surface 24 externe.

De préférence, le col 14 du récipient 10 comporte au moins une collerette 26 qui s'étend radialement vers l'extérieur et comporte une face 28 inférieure.

Le récipient 10 présente un axe O principal.

Tel qu'illustré sur les figures 1 et 2, l'axe O du récipient 10 s'étend ici verticalement selon le trièdre (L, V, T).

De préférence, les récipients 10 sont transportés dans une position dite « col en haut » lors de leur irradiation au défilé par au moins un faisceau (F) d'électrons délivré par le dispositif 100 de stérilisation.

Le dispositif 100 de stérilisation est par exemple intégré dans une installation (non représentée) de fabrication de récipients dans laquelle est ainsi réalisée la stérilisation par irradiation des récipients 10.

Une telle installation de fabrication de récipients 10, en particulier de bouteilles en PET, est bien connue de l'état de la technique.

La fabrication d'un récipient en matière thermoplastique y est obtenue à partir d'une préforme chaude, généralement préalablement conditionnée thermiquement dans une unité de conditionnement thermique ou four avant d'être introduite dans un des moules d'une unité de moulage (ou souffleuse) pour y être transformée par soufflage au moyen d'au moins un fluide sous pression, avec ou sans étirage.

La stérilisation des récipients est donc effectuée en aval de l'unité de soufflage ou souffleuse (non représentée) d'une telle installation de fabrication de récipients.

Dans une telle installation, les récipients 10 (et les préformes) en matière thermoplastique sont successivement transportés sous la forme d'un flux (fx) continu par un système 200 de convoyage à travers les différentes unités de l'installation assurant les étapes de fabrication.

Un tel système 200 de convoyage comporte en outre des roues de transfert dont de nombreuses réalisations sont connues.

On distingue pour mémoire principalement des roues de transfert comportant un plateau à encoches associé à des moyens de guidage et des roues de transfert comportant des pinces, commandées ou non au moins en ouverture.

La figure 1 représente ainsi un tronçon du parcours suivi par les récipients 10 provenant de l'unité de moulage, une flèche à chaque extrémité du tronçon indiquant le sens de circulation du flux (fx) de récipients 10, de l'amont vers l'aval.

Sur le tronçon du parcours de la figure 1, les récipients 10 du flux (fx) sont transportés successivement par des roues de transfert qui, ici au nombre de cinq, sont respectivement référencées 201 à 205.

Les récipients 10 sont transportés avec un écartement déterminé, dit pas P, correspondant à la distance entre les axes O de deux récipients 10 consécutifs du flux (fx).

Sur le tronçon du parcours associé au dispositif 100 de stérilisation, le pas P entre deux récipients 10 est constant, c'est-à-dire que le pas P ne varie pas entre la première roue 201 de transfert située en amont et la dernière roue 205 de transfert située en aval.

Selon une caractéristique importante, outre le pas P entre deux récipients 10 consécutifs, la vitesse de déplacement des récipients 10 formant ledit flux (fx) est également constante.

La vitesse de déplacement des récipients 10 détermine la cadence de production d'une telle installation de fabrication, exprimée en nombre de récipients par heure, ladite cadence étant notamment déterminée en fonction de l'unité de remplissage.

Le dispositif 100 de stérilisation comporte des émetteurs, ici cinq émetteurs référencés successivement 101 à 105, qui sont chacun destinés à irradier lesdits récipients 10 depuis l'extérieur avec un faisceau F d'électrons.

Les émetteurs 101 à 105 sont agencés le long du parcours suivi par le flux (fx) de récipients 10, un émetteur étant par exemple associé à chacune des roues de transfert 201 à 205.

Pour la mise en œuvre d'un procédé de traitement selon l'état de la technique, le dispositif 100 de stérilisation comporte un nombre important d'émetteurs nécessaires à l'obtention d'une dose d'irradiation finalement reçue par chaque récipient 10 qui permette d'en garantir la stérilisation.

Les émetteurs 101 à 105 sont agencés sur le côté du parcours suivi par le flux (fx) de récipients 10 de sorte que les récipients 10 sont successivement irradiés radialement depuis l'extérieur par chaque faisceau (F) d'électrons émis par l'un des émetteurs.

Chaque récipient 10 reçoit à chaque fois une dose d'irradiation donnée qui est notamment déterminée par la durée d'exposition audit faisceau (F) d'électrons, et donc par la vitesse de déplacement des récipients 10 devant les émetteurs 101 à 105.

Sur la figure 1, un récipient 10 du flux (fx) est successivement irradié par chacun des cinq émetteurs 101 à 105 du dispositif 100 de stérilisation et reçoit au final une quantité cumulée d'irradiation correspondant à une dose létale pour laquelle la stérilisation de sa surface 20 interne et de sa surface 24 externe du récipient 10 est obtenue.

Or, le coût d'un émetteur reste particulièrement élevé ce qui demeure un frein pour la stérilisation des récipients 10 par irradiation.

C'est l'une des raisons pour lesquelles, on recherche des solutions pour optimiser l'utilisation de tels émetteurs dans le but notamment de réduire le nombre total d'émetteurs utilisés, mais sans réduction des cadences de production.

Les émetteurs 101 à 105 formants ledit dispositif 100 de stérilisation sont identiques de sorte que la description de l'émetteur 101 réalisée ci-après vaut également pour les autres émetteurs du dispositif 100 de stérilisation.

Tel qu'illustré sur la figure 2, l'émetteur 101 présente un axe A principal qui s'étend verticalement selon le trièdre (L, V, T).

L'émetteur 101 comporte une tête 111 d'irradiation présentant une forme parallélépipédique et s'étendant, suivant son axe A principal, sur une hauteur « H » et orthogonalement audit axe A sur une largeur « l ».

Un émetteur 101 est ainsi susceptible d'irradier depuis l'extérieur, par l'intermédiaire de ladite tête 111 fixe, un récipient 10 présentant un diamètre externe D et verticalement une hauteur h, depuis son fond 16 jusqu'à son col 14, ladite hauteur h du récipient 10 étant inférieure ou égale à la hauteur H de la tête 111 de l'émetteur 101.

Cependant, si la hauteur H de la tête 111 de l'émetteur 101 est fixe, déterminée par construction, le format des récipients 10 varie lui en fonction des applications.

En effet, une même installation de fabrication est susceptible de fabriquer des récipients 10, tels que des bouteilles, allant par exemple d'une contenance d'une demi litre (ou moins) jusqu'à une contenance de deux litres.

Une partie du faisceau (F) d'électrons émis par la tête 111 de l'émetteur 101 est donc perdue dès lors qu'aucune partie du récipient 10 ne se trouve radialement en vis-à-vis pour être irradiée.

La partie perdue du faisceau (F) d'électrons émis par un émetteur est notamment fonction de la différence entre la hauteur H de la tête 111 et la hauteur h du récipient 10.

Cela constitue également un surcoût, au regard notamment de l'énergie électrique consommée pour alimenter l'émetteur produisant le faisceau (F) d'électrons.

Un émetteur 101 comporte une tête 111 ayant une hauteur H déterminée par construction tandis que la hauteur h des récipients 10 fabriqués varie quant à elle en fonction des applications.

Ainsi, lorsque le récipient 10 présente une hauteur h qui est égale à la moitié de la hauteur H de la tête de l'émetteur, la moitié du faisceau (F) d'électrons émis par la tête de l'émetteur est alors perdue puisque cette partie du faisceau (F) ne va irradier aucun récipient.

On décrira ci-après, par comparaison avec l'état de la technique illustré par les figures 1 et 2, un dispositif 100 de stérilisation par irradiation d'au moins un récipient 10 en matière thermoplastique.

Tel que décrit précédemment, le récipient 10 en matière thermoplastique est du type présentant un axe O principal et comportant un corps 12 muni d'un col 14 et fermé par un fond 16.

Le dispositif 100 de stérilisation comporte au moins un émetteur 120 qui, pourvu d'une tête 125, est réalisé selon l'invention.

Comme les émetteurs 101 à 105, l'émetteur 120 présente une tête 125 qui s'étend suivant un axe A principal et qui est destinée à émettre et à délimiter un faisceau (F) d'électrons pour irradier radialement au moins un récipient 10 depuis l'extérieur.

Selon l'invention, ledit au moins un émetteur 120 est configuré pour pouvoir faire pivoter la tête autour d'un axe de propagation du faisceau et ainsi incliner la tête 125 suivant un angle (α) d'inclinaison de l'axe A principal de l'émetteur par rapport à l'axe O du récipient. Ledit angle (α) est déterminé en fonction de la hauteur (h) du récipient 10.

Avantageusement, la valeur de l'angle (α) d'inclinaison est déterminée pour optimiser, maximiser, la dose d'irradiation reçue par un récipient 10 lorsque ledit récipient 10 est irradié par le faisceau (F) d'électrons émis par la tête 125 est que le récipient est déplacé par le système 200 de convoyage.

De préférence, l'angle (α) d'inclinaison de la tête 125 est déterminé de manière que le ratio de la hauteur (h) du récipient 10 sur la hauteur (H) de la tête 125 de l'émetteur soit proche de 1 pour limiter les pertes d'électrons dudit faisceau (F).

L'angle (α) d'inclinaison de la tête 125 est un angle aigu, situé « à l'intérieur » de l'intersection des axes A et O.

L'angle (α) d'inclinaison de la tête 125 est compris entre une valeur minimale supérieure à 0°, l'axe A de la tête 125 n'étant pas coaxial à l'axe O du récipient 10, et une valeur maximale de 90° correspondant à un agencement horizontal de la tête 125.

Par comparaison, tel qu'illustré à la figure 2, la tête 111 de l'émetteur 101 selon l'état de la technique est agencée de manière que l'axe A principal de l'émetteur s'étende verticalement, coaxialement à l'axe O du récipient 10.

Avantageusement, la tête 125 de l'émetteur 120 est montée mobile en rotation afin d'incliner sélectivement la tête 125 suivant une valeur donnée dudit angle (α) d'inclinaison.

De préférence, l'inclinaison de la tête 125 est commandée par des moyens d'actionnement (non représentés).

Avantageusement, la tête 125 est montée mobile en translation pour faire varier la distance entre la tête 125 et ledit au moins un récipient 10 à irradier, notamment en fonction du diamètre D externe du récipient 10.

De préférence, la tête 125 est montée télescopique par rapport à l'émetteur 120.

En variante non représentée, l'émetteur 120 est monté mobile en translation relativement audit au moins un récipient 10 à irradier, par exemple par l'intermédiaire d'un système de rails permettant de faire coulisser l'émetteur 120 et donc la tête 125 relativement au récipient 10.

Par comparaison à l'état de la technique, l'inclinaison de la tête 125 permet d'obtenir une augmentation de la dose d'électrons reçue par le récipient 10.

Avantageusement, une telle augmentation est susceptible de permettre une réduction du nombre d'émetteur(s) utilisé dans un dispositif 100 de stérilisation équipant une installation de fabrication de récipients 10 grâce à une exploitation du faisceau (F) d'électrons émis par la tête de l'émetteur.

Pour illustrer le principe de l'invention, on a représenté sur la figure 3 deux récipients 10 de dimensions différentes, un premier récipient 10 présentant une hauteur h1 et un deuxième récipient 10 présentant une hauteur h2, supérieure à la hauteur h1 du premier récipient.

Pour optimiser l'irradiation d'un récipient 10 et limiter les pertes d'électrons émis par un émetteur 120, la tête 125 de l'émetteur 120 est inclinée relativement au récipient 10 dudit angle (α) d'inclinaison déterminé en fonction de la hauteur h du récipient 10.

L'angle (α) d'inclinaison correspond à l'angle formé par l'intersection de l'axe A principal de l'émetteur 120 et l'axe O du récipient 10, la direction de déplacement suivi par le récipient 10 étant orthogonale à l'axe O principal du récipient 10, telle qu'illustrée par une flèche sur la figure 3.

Selon les deux exemples illustrés sur la figure 3, l'émetteur 120 est incliné suivant un angle (α1) d'inclinaison compris entre son axe A principal et l'axe O du récipient 10 de hauteur (h1) ou incliné suivant un angle (α2) d'inclinaison compris entre son axe A principal et l'axe O du récipient 10 de hauteur (h2).

De préférence, la tête 125 de l'émetteur 120 est inclinée de manière que les deux extrémités s'étendent respectivement au-delà du col 14 et du fond 16 du récipient 10, lesdites extrémités de l'émetteur 120 ayant été représentées sur la figure 3 par des rectangles en trait mixte.

Avantageusement, le col 14 et le fond 16 du récipient 10 sont ainsi irradiés de manière optimale par le faisceau (F) d'électrons émis par la partie de la tête 125 de l'émetteur 120 se trouvant radialement en vis-à-vis et non partiellement par une portion de celui-ci.

Tel qu'illustré sur la figure 3, le récipient 10 parcourt entre les extrémités de la tête 125 de l'émetteur 120 une distance L1 ou L2 en étant irradié successivement du fond 16 vers le col 14 (suivant le sens de déplacement du flux (fx) de la droite vers la gauche tel qu'indiqué par la flèche).

Avantageusement, le récipient 10 est simultanément entraîné en rotation sur lui-même, autour de son axe O, pendant son irradiation par le faisceau (F) d'électrons émis par l'émetteur 120.

De préférence, la vitesse d'entraînement en rotation sur lui-même du récipient 10 est déterminée pour que l'ensemble de la circonférence du récipient 10 soit irradiée au moins une fois.

Avantageusement, chaque récipient 10 est ainsi irradié de manière homogène par le faisceau (F) d'électrons (communément appelé « e-beam » en anglais).

De préférence, l'irradiation pour stériliser les récipients 10 depuis l'extérieur est obtenue au moyen d'un faisceau (F) d'électrons pulsé, c'est-à-dire un faisceau d'électrons qui est formé par une succession d'impulsions.

Avantageusement, les impulsions formant ledit faisceau (F) d'électrons pulsé présentent une durée d'émission, une intensité et une énergie qui sont déterminées en fonction des applications.

On pourra par exemple se reporter aux enseignements du document WO-2016/120544 précité sur les caractéristiques physiques d'un tel faisceau (F) d'électrons de type pulsé.

Avantageusement, un émetteur 120 comportant une tête 125 inclinable selon l'invention, est susceptible de remplacer au moins l'un des émetteurs 101 à 105 d'un dispositif 100 de stérilisation.

De préférence, plusieurs émetteurs 120 sont agencés le long du parcours suivi par le flux (fx) de récipients 10 transportés par le système 200 de convoyage.

Pour chaque type de récipient 10, le dispositif 100 de stérilisation est alors susceptible d'être optimisé en inclinant la tête de chaque émetteur pour en exploiter au maximum le faisceau (F) d'électrons émis.

Avantageusement, le nombre total d'émetteurs nécessaires à l'obtention de la dose d'irradiation d'un récipient 10 donné est susceptible d'être réduit dès lors que les pertes d'électrons sont complètement supprimées par comparaison avec l'état de la technique décrit en référence aux figures 1 et 2.

Ainsi, il est par exemple possible d'utiliser un nombre moindre d'émetteur(s), par exemple trois émetteurs contre cinq auparavant, et cela sans pour autant que la dose d'irradiation reçue par le récipient 10 ne s'en trouve affectée, préservant ainsi le degré de stérilisation finalement obtenu.

Avantageusement, la réduction du nombre d'émetteur(s) dans un dispositif 100 de stérilisation permet de réduire de manière substantielle les coûts de mise en œuvre d'une stérilisation par irradiation au moyen de faisceau (F) d'électrons.

En effet, pour un résultat de stérilisation au moins équivalent, la réduction du nombre d'émetteur(s) permet tout d'abord de réduire le coût d'acquisition d'un dispositif 100 de stérilisation, notamment destiné à équiper une installation de fabrication de récipients 10.

Avantageusement, la réduction du nombre d'émetteur(s) permet ensuite de réduire aussi les coûts d'exploitation d'un dispositif 100 de stérilisation, en particulier la consommation d'énergie électrique nécessaire pour produire un faisceau (F) d'électrons.

Le dispositif 100 de stérilisation comporte plusieurs émetteurs 120, par exemple trois, qui sont agencés le long du parcours suivi par le flux (fx) de récipients 10 transportés par le système 200 de convoyage tel qu'illustré sur la figure 1.

Un plan de déplacement est défini par la direction de l'axe (O) principal des récipients et par la direction de déplacement suivi par le flux (fx) de récipients 10.

Un plan d'irradiation est défini par la direction de propagation du faisceau et par la direction de l'axe (A) principal de la tête.

Avantageusement, le plan de déplacement et le plan d'irradiation sont perpendiculaires l'un par rapport à l'autre.

Dans un premier mode de réalisation avantageux, la direction de propagation est perpendiculaire au plan de déplacement, l'angle (α) sert à optimiser l'irradiation au cours de déplacement du récipient.

Dans un second mode de réalisation avantageux, la direction de propagation n'est pas perpendiculaire au plan de déplacement. On tient compte de la hauteur du récipient pour positionner la tête de manière à irradier le fond du récipient et ainsi permettre l'optimisation de l'irradiation.

L'invention propose encore un procédé de stérilisation par irradiation d'un tel flux (fx) de récipients 10 en matière thermoplastique du type présentant comme précédemment un axe (O) principal et comportant un corps 12 muni d'un col 14 et fermé par un fond 16 par un dispositif 100 de stérilisation comportant au moins un émetteur 120 pourvu d'une tête 125 qui, présentant un axe (A) principal, est destinée à émettre un faisceau (F) d'électrons.

Comme précédemment, la direction de déplacement suivi par le flux (fx) de récipients 10 transportés par le système 200 de convoyage est orthogonale à l'axe (O) principal des récipients 10.

Selon l'invention, ledit procédé de traitement comporte au moins une étape d'irradiation dudit au moins récipient 10 depuis l'extérieur par un faisceau (F) d'électrons émis par la tête 125 dudit émetteur 120 agencée pour présenter un angle (α) d'inclinaison qui, compris entre l'axe (A) principal de l'émetteur et l'axe (O) du récipient, est égal à une valeur déterminée en fonction de la hauteur (h) du récipient 10.

Avantageusement, le procédé de traitement comporte au moins une étape préliminaire de réglage consistant, lorsque la direction de déplacement suivi par le flux (fx) de récipients 10 est orthogonale à l'axe (O) principal des récipients 10, à régler l'inclinaison de la tête 125 dudit émetteur 120 suivant un angle (α) d'inclinaison d'une valeur déterminée pour que le ratio de la hauteur (h) du récipient 10 sur la hauteur (H) de la tête 125 de l'émetteur 120 soit proche de 1.

Pour augmenter encore la dose d'irradiation reçue par chaque récipient 10 du flux (fx), l'écartement entre deux récipients 10 consécutifs supérieur à la largeur « l » de l'émetteur 101 illustré sur la figure 2 est avantageusement réduit de manière qu'un récipient 10 se trouve toujours en vis-à-vis du faisceau (F) d'électrons.

Tel qu'expliqué en référence à la figure 1, le flux (fx) de récipients 10 est transporté par le système 200 de convoyage suivant un parcours donné, avec un écartement déterminé, dit pas (P) initial, correspondant à la distance entre les axes (O) de deux récipients (10) consécutifs.

Avantageusement, le procédé de traitement comporte au moins une étape de commande du système de convoyage pour faire varier sélectivement ledit pas (P) initial entre deux récipients 10 consécutifs afin de réduire, au moins dans une zone d'irradiation du parcours dans laquelle est agencé ledit au moins émetteur 120 comportant la tête 125, l'écartement entre les récipients 10 dudit flux (fx) à un pas (P') proximal qui est inférieur audit pas (P) initial.

Avantageusement, la modification dudit pas (P) initial pour obtenir le pas (P') proximal entre les récipients 10 dudit flux (fx) est obtenue en faisant varier la vitesse de déplacement des récipients 10 transportés au voisinage de ladite zone d'irradiation.

La variation du pas est par exemple obtenue par des moyens mécaniques de type à came et galet ou préférentiellement par des moyens électriques, tels que des moteurs, associés à chaque récipient 10 transporté.

L'étape de commande du système de convoyage comporte une étape initiale de modification du pas (P) qui comporte au moins une phase de décélération des récipients 10 afin d'obtenir ledit pas (P') proximal.

Le pas (P') proximal correspond à un écartement minimal pour lequel les récipients 10 juxtaposés sont aptes à être entraînés en rotation sur eux-mêmes sans interférence, sans contact.

Avantageusement, la réduction du pas (P) est obtenue par une réduction de la vitesse des récipients passant en vis-à-vis de la tête de l'émetteur grâce à quoi le temps d'exposition au faisceau (F) d'électrons est augmentée, soit finalement la dose d'irradiation reçue par le récipient 10 correspondant à une dose létale.

De préférence, la vitesse des récipients 10 est réduite mais reste supérieure à zéro. En variante, la vitesse des récipients 10 pourrait toutefois être temporairement nulle, en marquant un arrêt lors de l'irradiation du récipient par l'émetteur.

Avantageusement, l'étape de commande du système de convoyage comporte une étape finale de modification du pas comportant au moins une phase d'accélération pour faire varier à nouveau l'écartement des récipients 10 afin de rétablir, après l'irradiation, ledit pas (P) initial entre les récipients 10 du flux (fx).

## Revendications

1. Dispositif (100) de stérilisation par irradiation d'au moins un récipient (10) en matière thermoplastique du type présentant un axe (O) principal et comportant un corps (12) muni d'un col (14) et fermé par un fond (16), ledit dispositif (100) comportant :
- un système de convoyage présentant une direction de déplacement du récipient et apte à transporter le récipient dont l'axe (O) principal est orthogonal à la direction de déplacement,
- au moins un émetteur (120) pourvu d'une tête (125) apte à émettre un faisceau (F) d'électrons pour irradier depuis l'extérieur ledit au moins un récipient (10) selon une direction de propagation du faisceau, la tête (125) présentant un axe A principal orthogonal à la direction de propagation du faisceau,
**caractérisé en ce que** ledit au moins un émetteur (120) comprend une tête (125) d'irradiation présentant une forme parallélépipédique et s'étendant, suivant son axe A principal, sur une hauteur « H » et orthogonalement audit axe A sur une largeur « l » de manière à émettre et à délimiter un faisceau (F) d'électrons pour irradier radialement au moins un récipient (10) depuis l'extérieur, et **en ce que** ledit émetteur (120) est configuré pour pouvoir incliner la tête (125) suivant un angle (α) d'inclinaison qui est compris entre l'axe (A) principal de la tête de l'émetteur et l'axe (O) du récipient et qui est déterminé en fonction de la hauteur (h) du récipient afin d'optimiser la dose d'irradiation reçue par ledit récipient (10).

2. Dispositif selon la revendication 1, **caractérisé en ce que** la tête (125) délimite une section droite du faisceau (F) et présente une hauteur (H) correspondant à la plus grande dimension de la section droite, le dispositif présente une hauteur projetée (H1) égale à la projection de la hauteur de la tête sur une direction orthogonale à la direction de déplacement, l'angle (α) d'inclinaison de la tête (125) est déterminé de manière que un ratio d'une hauteur (h) du récipient sur la hauteur projetée (H1) de l'émetteur soit proche de 1.

3. Dispositif selon la revendication 1 ou 2, **caractérisé en ce que** l'angle (α) d'inclinaison de la tête (125) est compris entre une valeur minimale supérieure à 0° et une valeur maximale de 90°.

4. Dispositif selon l'une quelconque des revendications 1 à 3, **caractérisé en ce que** la tête (125) de l'émetteur (120) est montée mobile en rotation afin d'incliner la tête (125) suivant ledit angle (α) d'inclinaison.

5. Dispositif selon la revendication 4, **caractérisé en ce que** l'inclinaison de la tête (125) est commandée par des moyens d'actionnement.

6. Dispositif selon l'une quelconque des revendications précédentes, **caractérisé en ce que** la tête (125) est montée mobile en translation pour faire varier la distance entre la tête (125) et ledit au moins un récipient (10) à irradier.

7. Procédé de stérilisation par irradiation d'un flux (fx) de récipients (10) en matière thermoplastique du type présentant un axe (O) principal et comportant un corps (12) muni d'un col (14) et fermé par un fond (16) par un dispositif (100) de stérilisation comportant au moins un émetteur (120) pourvu d'une tête (125) qui, présentant un axe (A) principal, est destinée à émettre un faisceau (F) d'électrons, le procédé comprenant :
- une étape d'irradiation dudit au moins récipient (10) depuis l'extérieur par le faisceau (F) d'électrons émis par la tête (125) dudit émetteur (120) agencée pour présenter un angle (α) d'inclinaison qui, compris entre l'axe (A) principal de la tête de l'émetteur et l'axe (O) du récipient, est égal à une valeur déterminée en fonction de la hauteur (h) du récipient le long de l'axe (O) principal du récipient, ladite tête (125) d'irradiation présentant une forme parallélépipédique et s'étendant, suivant son axe A principal, sur une hauteur « H » et orthogonalement audit axe A sur une largeur « l » de manière à émettre et à délimiter un faisceau (F) d'électrons pour irradier radialement au moins un récipient (10) depuis l'extérieur.

8. Procédé de traitement selon la revendication 7, **caractérisé en ce que** ledit procédé de traitement comporte au moins :
- une étape de transport du flux de récipients le long d'une direction de déplacement,
- calculer une hauteur (h1) projetée du récipient sur une direction orthogonale à la direction de déplacement,
- la tête (125) délimite une section droite du faisceau (F) présentant une hauteur (H) correspondant à la plus grande dimension de la section droite, calculer une hauteur (H1) projetée égale à la projection de la hauteur (H) de la tête sur la direction orthogonale à la direction de déplacement,
- une étape de réglage préliminaire à l'étape d'irradiation consistant, à régler l'inclinaison de la tête (125) dudit émetteur (120) suivant un angle (α) d'inclinaison d'une valeur déterminée pour qu'un ratio de la hauteur projetée (h1) du récipient sur la hauteur projetée (H1) de la tête de l'émetteur soit proche de 1.

## Patentansprüche

1. Vorrichtung (100) zur Sterilisation durch Bestrahlung mindestens eines Behälters (10) aus thermoplastischem Material des Typs, der eine Hauptachse (O) aufweist und einen Körper (12) umfasst, der mit einem Hals (14) versehen ist und durch einen Boden (16) verschlossen wird, die Vorrichtung (100) umfassend:
- ein Fördersystem, das eine Verlagerungsrichtung des Behälters aufweist und geeignet ist, den Behälter, dessen Hauptachse (O) orthogonal zu der Verlagerungsrichtung verläuft, zu transportieren,
- mindestens einen Emitter (120), der mit einem Kopf (125) ausgestattet ist, der geeignet ist, einen Elektronenstrahl (F) zu emittieren, um den mindestens einen Behälter (10) von außen entlang einer Ausbreitungsrichtung des Strahls zu bestrahlen, wobei der Kopf (125) eine Hauptachse A aufweist, die orthogonal zu der Ausbreitungsrichtung des Strahls verläuft,
**dadurch gekennzeichnet, dass** der mindestens eine Emitter (120) einen Bestrahlungskopf (125) umfasst, der eine parallelepipedische Form aufweist und sich entlang seiner Hauptachse A über eine Höhe "H" und orthogonal zu der Achse A über eine Breite "l" erstreckt, so dass er einen Elektronenstrahl (F) emittiert und begrenzt, um radial mindestens einen Behälter (10) von außen zu bestrahlen, und dadurch, dass der Emitter (120) dazu ausgestaltet ist, den Kopf (125) gemäß einem Neigungswinkel (α) neigen zu können, der zwischen der Hauptachse (A) des Kopfes des Emitters und der Achse (O) des Behälters liegt und der in Abhängigkeit von der Höhe (h) des Behälters bestimmt wird, um die von dem Behälter (10) aufgenommene Bestrahlungsdosis zu optimieren.

2. Vorrichtung nach Anspruch 1, **dadurch gekennzeichnet, dass** der Kopf (125) einen Querschnitt des Strahls (F) begrenzt und eine Höhe (H) aufweist, die dem größten Maß des Querschnitts entspricht, die Vorrichtung eine projizierte Höhe (H1) gleich der Projektion der Höhe des Kopfes in einer zu der Verlagerungsrichtung orthogonalen Richtung aufweist, der Neigungswinkel (α) des Kopfes (125) so bestimmt wird, dass ein Verhältnis einer Höhe (h) des Behälters zu der projizierten Höhe (H1) des Emitters nahe 1 ist.

3. Vorrichtung nach Anspruch 1 oder 2, **dadurch gekennzeichnet, dass** der Neigungswinkel (α) des Kopfes (125) zwischen einem minimalen Wert größer 0° und einem maximalen Wert von 90° liegt.

4. Vorrichtung nach einem der Ansprüche 1 bis 3, **dadurch gekennzeichnet, dass** der Kopf (125) des Emitters (120) drehbeweglich montiert ist, um den Kopf (125) gemäß dem Neigungswinkel (α) zu neigen.

5. Vorrichtung nach Anspruch 4, **dadurch gekennzeichnet, dass** das Neigen des Kopfes (125) durch Betätigungsmittel gesteuert wird.

6. Vorrichtung nach einem der vorhergehenden Ansprüche, **dadurch gekennzeichnet, dass** der Kopf (125) translatorisch beweglich montiert ist, um den Abstand zwischen dem Kopf (125) und dem mindestens einen zu bestrahlenden Behälter (10) variieren zu lassen.

7. Verfahren zur Sterilisation durch Bestrahlung eines Stroms (fx) von Behältern (10) aus thermoplastischem Material des Typs, der eine Hauptachse (O) aufweist und einen Körper (12) umfasst, der mit einem Hals (14) versehen ist und durch einen Boden (16) verschlossen wird, durch eine Vorrichtung (100) zur Sterilisation, die mindestens einen Emitter (120) umfasst, der mit einem Kopf (125) ausgestattet ist, der, eine Hauptachse (A) aufweisend, dazu bestimmt ist, einen Elektronenstrahl (F) zu emittieren, wobei das Verfahren umfasst:
- einen Schritt des Bestrahlens des mindestens einen Behälters (10) von außen mit dem Elektronenstrahl (F), der von dem Kopf (125) des Emitters (120) emittiert wird, der so angeordnet ist, dass er einen Neigungswinkel (α) aufweist, der zwischen der Hauptachse (A) des Kopfes des Emitters und der Achse (O) des Behälters liegt und gleich einem in Abhängigkeit von der Höhe (h) des Behälters entlang der Hauptachse (O) des Behälters bestimmten Wert ist, wobei der Bestrahlungskopf (125) eine parallelepipedisch Form aufweist und sich entlang seiner Hauptachse A über eine Höhe "H" und orthogonal zu der Achse A über eine Breite "l" erstreckt, so dass er einen Elektronenstrahl (F) emittiert und begrenzt, um mindestens einen Behälter (10) radial von außen zu bestrahlen.

8. Verfahren zur Behandlung nach Anspruch 7, **dadurch gekennzeichnet, dass** das Verfahren zur Behandlung mindestens umfasst:
- einen Schritt des Transportierens des Stroms von Behältern entlang einer Verlagerungsrichtung,
- Berechnen einer projizierten Höhe (h1) des Behälters in einer orthogonal zu der Verlagerungsrichtung verlaufenden Richtung,
- der Kopf (125) einen Querschnitt des Strahls (F) begrenzt, der eine Höhe (H) aufweist, die dem größten Maß des Querschnitts entspricht, Berechnen einer projizierten Höhe (H1) gleich der Projektion der Höhe (H) des Kopfes in einer orthogonal zu der Verlagerungsrichtung verlaufenden Richtung,
- einen Schritt des Einstellens im Vorfeld des Bestrahlungsschritts, der darin besteht, die Neigung des Kopfes (125) des Emitters (120) gemäß einem Neigungswinkel (α) mit einem bestimmten Wert einzustellen, damit ein Verhältnis der projizierten Höhe (h1) des Behälters zur projizierten Höhe (H1) des Kopfes des Emitters nahe 1 ist.

## Claims

1. Device (100) for sterilizing by irradiation at least one container (10) made of thermoplastic material of the type having a main axis (O) and comprising a body (12) provided with a neck (14) and closed by a bottom (16), said device (100) comprising:
- a conveying system having a direction of displacement of the container and capable of transporting the container of which the main axis (O) is orthogonal to the direction of displacement,
- at least one emitter (120) provided with a head (125) capable of emitting a beam (F) of electrons to irradiate from the outside said at least one container (10) in a direction of propagation of the beam, the head (125) having a main axis A orthogonal to the direction of propagation of the beam,
**characterized in that** said at least one emitter (120) comprises an irradiation head (125) having a parallelepipedal form and extending, along its main axis A, over a height "H" and orthogonally to said axis A over a width "l" so as to emit and to delimit a beam (F) of electrons to radially irradiate at least one container (10) from the outside, and **in that** said emitter (120) is configured to be able to tilt the head (125) according to a tilt angle (α) which lies between the main axis (A) of the head of the emitter and the axis (O) of the container and which is determined as a function of the height (h) of the container in order to optimize the irradiation dose received by said container (10).

2. Device according to Claim 1, **characterized in that** the head (125) delimits a cross section of the beam (F) and has a height (H) corresponding to the greatest dimension of the cross section, the device has a projected height (H1) equal to the projection of the height of the head on a direction orthogonal to the direction of displacement, the tilt angle (α) of the head (125) is determined such that a ratio of a height (h) of the container to the projected height (H1) of the emitter is close to 1.

3. Device according to Claim 1 or 2, **characterized in that** the tilt angle (α) of the head (125) lies between a minimum value greater than 0° and a maximum value of 90°.

4. Device according to any one of Claims 1 to 3, **characterized in that** the head (125) of the emitter (120) is mounted so as to be able to move in rotation in order to tilt the head (125) according to said tilt angle (α).

5. Device according to Claim 4, **characterized in that** the tilt of the head (125) is controlled by actuation means.

6. Device according to any one of the preceding claims, **characterized in that** the head (125) is mounted so as to be able to move in translation to vary the distance between the head (125) and said at least one container (10) to be irradiated.

7. Method for sterilizing by irradiation a flow (fx) of containers (10) made of thermoplastic material of the type having a main axis (O) and comprising a body (12) provided with a neck (14) and closed by a bottom (16) by a sterilization device (100) comprising at least one emitter (120) provided with a head (125) which, having a main axis (A), is intended to emit a beam (F) of electrons, the method comprising:
- a step of irradiation of said at least one container (10) from the outside by the beam (F) of electrons emitted by the head (125) of said emitter (120), which is arranged to have a tilt angle (α) which, lying between the main axis (A) of the head of the emitter and the axis (O) of the container, is equal to a value determined as a function of the height (h) of the container along the main axis (O) of the container, said irradiation head (125) having a parallelepipedal form and extending, along its main axis A, over a height "H" and orthogonally to said axis A over a width "l" so as to emit and to delimit a beam (F) of electrons to radially irradiate at least one container (10) from the outside.

8. Treatment method according to Claim 7, **characterized in that** said treatment method comprises at least:
- a step of transporting the flow of containers along a direction of displacement,
- calculating a projected height (h1) of the container on a direction orthogonal to the direction of displacement,
- the head (125) delimits a cross section of the beam (F) having a height (H) corresponding to the greatest dimension of the cross section, calculating a projected height (H1) equal to the projection of the height (H) of the head on the direction orthogonal to the direction of displacement,
- a setting step preliminary to the irradiation step, consisting in setting the tilt of the head (125) of said emitter (120) according to a tilt angle (α) of a value that is determined so that a ratio of the projected height (h1) of the container to the projected height (H1) of the head of the emitter is close to 1.
